# EUROPEAN PATENT SPECIFICATION

(11) **EP 2 138 090 B1**
(45) Date of publication and mention of the grant of the patent: **28.09.2011**
(21) Application number: 09007956.7
(22) Date of filing: 17.06.2009
(51) Int. Cl.: A61B 1/005, A61B 1/008

(54) **Endoscope**
Endoskop
Endoscope

(30) Priority: 23.06.2008 JP 2008162772
(43) Date of publication of application: 30.12.2009
(73) Proprietor: FUJIFILM Corporation, Minato-ku Tokyo (JP)
(72) Inventor: Torii, Yuuichi, Kita-ku Saitama-shi Saitama (JP)
(74) Representative: Klunker . Schmitt-Nilson . Hirsch

(56) References cited:
- EP-A- 0 313 836
- EP-A- 1 854 398
- WO-A-2004/011076
- US-A1- 2004 082 883
- US-A1- 2006 015 009

## Description

### [TECHNICAL FIELD]

This invention relates to an endoscope for medical use, and more particularly to a medical endoscope which can be inserted into and extracted out of a body cavity smoothly even through a constricted part or parts which may exist en route of an insertion passage.

### [TECHNICAL BACKGROUND]

In general, a medical endoscope is largely composed of a hand-held head grip with manual control means to be manipulated by an operator, and an insertion tube extended forward from the manipulating head grip for insertion into a body cavity. From a proximal end which is connected to the manipulating head grip, the insertion tube is mostly occupied by a flexible tube section which is bendable in arbitrary directions along a path of insertion. Relatively short articular section and rigid tip end section are successively attached to a fore distal end of the flexible tube section. At least an illumination window or windows and an observation window of an optical observation system are provided in a housing of the rigid tip end section. Further, in many cases, an exit opening of an instrument introduction channel, for introduction of forceps and other instruments, is provided in the housing of the rigid tip end section, along with an injection nozzle for spurting a cleaning fluid toward the observation window. The articular section intervening between the flexible tube section and the rigid tip end section can be flexed by remote control from the manipulating head grip to turn the rigid tip end section in a desired direction.

In upper endoscopy, it has been the general practice to insert a flexible tube of an endoscope into the body of an examinee by way of the oral cavity. However, recently there are endoscopes which are designed to be inserted by way of the nasal cavity. In case an endoscopic insertion tube is introduced by way of the nasal cavity, there is no possibility of irritating the root of the tongue. Besides, less stimulation is given to the examinee upon entrance to the alimentary canal, so that vomiting sensations as well as pains and burdens on the part of the examinee can be lessened to a considerable degree. In addition, the examinee can talk with the operator during an endoscopic examination, and can breathe through the mouth.

In the case of a per nasal insertion route, an endoscopic insertion tube is introduced through the nostril, the entrance to the nasal cavity, and then into a narrow passage in the nasal cavity including a middle and inferior passage toward the esophagus via the choana and pharynx. In this route, the insertion passage is most constricted at a gap which is formed by the nasal septum and middle and inferior turbinates or concha. In order to permit insertion through such a constricted part in the nasal cavity, there have been developed endoscopes having a narrow insertion tube, for example, as narrow as approximately from 5mm to 6m in diameter. However, structural parts of insertion passages through the nasal cavity vary largely from examinee to examinee, and the above-mentioned constricted part also varies from examinee to examinee in dimension. An endoscopic insertion tube can be passed through a narrow part of a passage through the nasal cavity by advancing same in such a way as to push and spread apart the superior, middle or inferior turbinates to a certain degree.

Generally, the outside diameter of an endoscopic insertion tube mainly depends upon dimensions of the articular section, which in some cases can be flexed up to a maximum angle of 180 degrees or more by manipulation of a flexion control means on the manipulating head grip, either in two directions including upward and downward directions or in four directions including upward, downward, rightward and leftward directions. In general, an articular section on endoscopic insertion tubes is constituted by an articulated ring structure consisting of a series of flexing rings which are successively connected to each other by the use of pivot pins. The articular section is required to be able to retain a predetermined shape even when it is flexed to a maximum angle. Therefore, relatively thick strong metal rings are used in forming the articulated structure. A plural number of manipulation wires from a flexion control means are connected to an articular section for flexing same in a desired direction. Such manipulation wires need to be retained in predetermined radial positions within the articular section of the insertion tube. For this purpose, it is the usual practice to provide wire threading slits on an articulated ring, cylindrical wire guide rings or wire threading eyelets on the pivot pins at predetermined radial positions. A number of parts such as a light guide, a signal line cable, a tube of a tool deliver channel and an air/water feed tube are also threaded internally of the endoscopic insertion tube. Of these threaded parts, the light guide and signal line cable are fragile, so that it is desirable to lower a packing rate and secure an ample extra room internally of the insertion tube for the purpose of preventing breakage of fragile members which might occur when in a pressed or twisted state.

The outside diameter of the articular section of an endoscopic insertion tube is determined in the first place in consideration of the requirements as discussed above. Thus, it is the general practice to determine outside diameters of rigid tip end section and flexible tube section of the insertion tube afterwards on the basis of the dimensions of the articular section. Nevertheless, the rigid tip end section which is connected to the fore distal end of the articular section is not necessarily required to be formed in the same diameter as the articular section. The rigid tip end section can be formed in a smaller diameter or in a forwardly tapered shape depending upon the layout of illumination and observation windows which are provided on a distal end face of the rigid tip end section in correlated positions with an outlet opening of a tool deliver channel and a wash fluid jet nozzle. Particularly, in case the rigid tip end section is in a forwardly tapered shape, it can positively perform a function of spreading wider a constricted part which may exist en route of an insertion passage, improving the maneuverability in an endoscope insertion process in such a way as to lessen the burdens on the part of an examinee, as disclosed in Japanese Patent Application laid open under 2007-301083.

In the case of the endoscope of the prior art just mentioned, although a rigid tip end section of an insertion tube is shaped in a forwardly tapered form, the endoscope is provided with a flexible tube section of the same diameter as the proximal end of the rigid tip end section. Therefore, as the insertion tube is put into the nasal cavity, not alone the rigid tip end section but the elongated flexible tube section acts to spread a constricted passage surrounded by the nasal septum and middle and inferior turbinates, arousing oppressive sensations on the side of the examinee all the time even after the articular section has passed the constricted part. Thus, in order to lessen the burdens on the part of an examinee, it is desirable to reduce the outside diameter of each section of an endoscopic insertion tube as much as possible irrespective of an outside diameter of other section which is built in predetermined dimensions, rather than forming various sections of an insertion tube uniformly in the same diameter.

The elongated flexible tube section which is connected to a proximal end of an articular section of an endoscopic insertion tube suffices to be bendable relatively moderately. As compared with the articular section, the flexible tube section is not required to have high strength in bending directions. Generally, the flexible tube section employs, as a flexible structural member, a coil tube which is composed of a couple of thin coil tubes fitted one in the other. Further, the flexible tube section can have a higher packing rate or density as compared with the articular section. The articular section of the endoscopic insertion tube needs to be covered with an elastic outer skin later which is formed of a resilient material like rubber. More specifically, a mesh tube and then an outer skin layer of rubber or the like is fitted on an articulated ring structure of the articular section. Each axial end of the outer skin layer is fixed in position by line wrapping and a cementing adhesive agent of a fastener means. On the other hand, in the flexible tube section, a mesh tube is fitted on the flexible coil tube and then an outer skin layer is integrally laminated on the mesh tube.

By arranging an endoscopic insertion tube in the manner as described above, the elongated flexible tube section can be formed in a reduced diameter as compared with the outside diameter of the articular section. A reduction in outside diameter of the flexible tube section can contributes to reduce burdens on the side of an examinee under endoscopic examination. However, in case the flexible tube section is formed in a smaller diameter than the articular section, the circumference of the flexible tube section is discontinued from that of the articular section by a step of a height which would be increased in proportion to a difference in outside diameter of the flexible tube section and the articular section. Therefore, when the endoscopic insertion tube is introduced into the nasal cavity, a constricted portion en route of an insertion passage is once spread wide by the articular section of the insertion tube and then allowed to return to an original constricted state as soon as the narrower flexible tube section reaches there, depending upon the difference in outside diameter between the difference in outside diameter. As a consequence, at the time of extracting the insertion tube out of the nasal cavity, the stepped portion between the articular and flexible tube sections would be caught in the constricted portion. In that case, it becomes difficult to extract the insertion tube without causing great pains on the part of an examinee.

### [SUMMARY OF THE INVENTION

With the foregoing situations in view, it is an object of the present invention to provide an endoscope employing an insertion tube which is arranged to have a largest outside diameter in an articular section between an elongated flexible tube section and a rigid tip end section, the insertion tube being gradually tapered off axially in both forward and inverse directions from the articular section to ensure improved maneuverability when passing the insertion tube through a constricted part of an insertion passage in the nasal cavity.

It is another object of the present invention to provide an endoscope which is equipped with an insertion tube having a maximum diameter portion in an articular section between a rigid tip end section and an elongated flexible tube section in such a way as to ensure smooth passage to and from the rigid tip end section and the flexible tube section at a constricted portion in an insertion passage.

According to the present invention, in order to achieve the above-stated objectives, there is provided an endoscope having an insertion tube extended out of a manipulating head grip, the insertion tube having in series an elongated flexible tube section, an articular section and a rigid tip end section from a proximal end connected to the manipulating head grip, characterized in that the endoscope comprises: a rigid tip end section tapered off in a forward direction gradually toward a fore distal end from a proximal end connected with the articular section and provided with at least an illumination window and an observation window on a fore distal end face; an articular section having a larger outside diameter than any other section of the insertion tube; and a transitional section tapered off in an inverse direction as compared with the rigid tip end section, gradually from a fore end on the side of the articular section toward a proximal end on the side of the flexible tube section.

Medical endoscopes for use in upper digestive tract endoscopy are classified into a peroral type and a per-nasal type depending on the route of insertion. Particularly, the per-nasal insertion route contains an extremely narrow constricted part en route of an insertion passage. Although the endoscope according to the present invention is suitable for use as a per-nasal endoscope, its use is not limited to a per-nasal route and can also be applied to other routes of insertion.

The articular section as well as the elongated flexible tube section and the rigid tip end section have a peculiar or unique construction different from other sections. The rigid tip end section is gradually tapered off from its proximal end which is joined with the articular section, toward its fore distal end face on which illumination windows and an endoscopic observation window are provided. When the rigid tip end section is arranged in this way, it can be advanced smoothly through an insertion passage, even through a narrow constricted part which may exist en route of the insertion passage, by spreading wide the constricted part by a tapered body. After passage of the thickest articular section, what remains in the constricted part of the insertion passage is the flexible tube section which is reduced in diameter to lessen oppressive sensations and burdens on the part of an examinee.

The flexible tube section is appreciably reduced in diameter as compared with the articular section, and joined with the latter through a tapered transitional section thereby to eliminate a stepped surface which would otherwise come to exist due to the difference in diameter between the articular and flexible tube sections. In this regard, it is possible to provide such a transitional section by gradually tapering off an end portion of the articular section or an end portion of the flexible tube section. In a preferred form of the invention, the flexible tube section which is uniform in outside diameter in the axial direction is joined with the articular section through a rigid taper ring which is gradually tapered off from a fore end on the side of the articular section toward a proximal end on the side of the flexible tube section. From the standpoint of maneuverability of the insertion tube, the taper ring should have a moderate taper angle. However, since the taper ring is a rigid ring, there is a limit to reductions of the taper angle to avoid elongation of the rigid part to an objectionable degree.

The transitional section is tapered in an inverse direction as compared with the forwardly tapered rigid tip end section. The taper angle of the inversely tapered transitional section may same as or akin to that of the forwardly tapered rigid tip end section. An outer skin layer which is fitted on the articular section is fixed at opposite ends by a fastener means composed of line wrapping and an adhesive agent. The fastener means, including the adhesive agent, is applied in such a way as to present a tapered shape in conformity with the profile of the tapered ring.

The above and other objects, features and advantages of the invention will become apparent from the following particular description of the invention, taken in conjunction with the accompanying drawings which show by way of example a preferred embodiment of the invention. Needless to say, the present invention should not be construed as being limited to particular forms shown in the drawings.

### [BRIEF DESCRIPTION OF THE DRAWINGS]

In the accompanying drawings:
Fig. 1 is a schematic illustration, showing the general layout of an endoscope embodying the present invention;
Fig. 2 is a longitudinal sectional view of a fore end portion of an endoscopic insertion tube;
Fig. 3 is an enlarged longitudinal sectional view of a fore distal end portion of the insertion tube, from a distal to proximal end of a rigid tip end section which is joined with an articular section of the insertion tube;
Fig. 4 is an enlarged longitudinal sectional view of a transitional section intervening between the articular section and the flexible tube section of the insertion tube, with some internally threaded parts omitted for the convenience of illustration;
Fig. 5 is a front view of the rigid tip end section of the insertion tube; and
Fig. 6 is a sectional view taken in the direction of arrow X-X in Fig. 2.

### [PREFERRED EMBODIMENT(S) OF THE INVENTION]

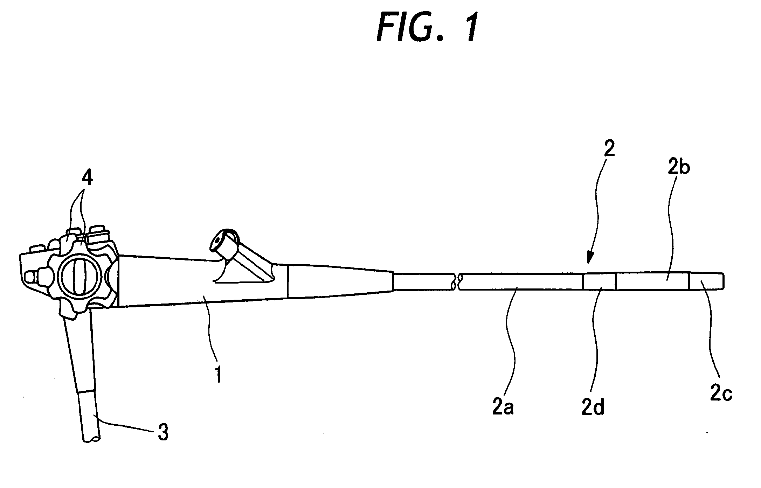

Hereafter, the present invention is described more particularly by way of its preferred embodiments. Shown schematically in Fig. 1 is the general layout of an endoscope embodying the present invention. As seen in Fig. 1, the endoscope is largely constituted by a manipulating head grip 1, an insertion tube 2 to be introduced into a body cavity, and a universal connection cable 3. From a proximal end which is connected to the manipulating head grip 1, the endoscopic insertion tube 2 is mostly occupied by an elongated flexible tube section 2a, which is ensued by relatively short articular section 2b and rigid tip end section 2c. The articular section 2b can be flexed by remote control from the manipulating head grip 1 to turn the rigid tip end section 2c to an arbitrary direction. More particularly, the articular section 2b is flexed by manipulation of a flexion control means 4 which is provided on the manipulating head grip 1. In the case of the particular embodiment shown, the endoscope incorporates a two-way flexion mechanism to flex the articular section in two directions, i.e., in upward and downward directions. However, if necessary, the endoscope can incorporate 4-way flexion mechanism to flex the articular section 2b in four directions, i.e., in upward, downward, rightward and leftward directions.

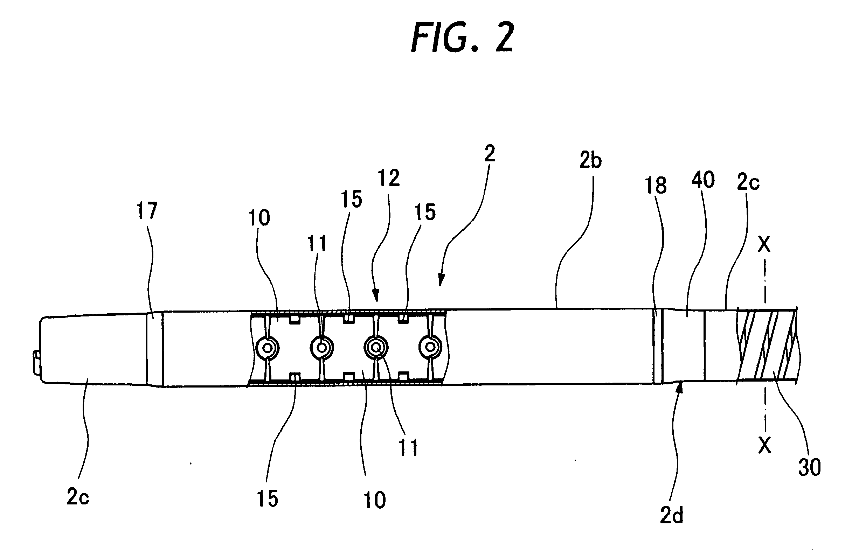

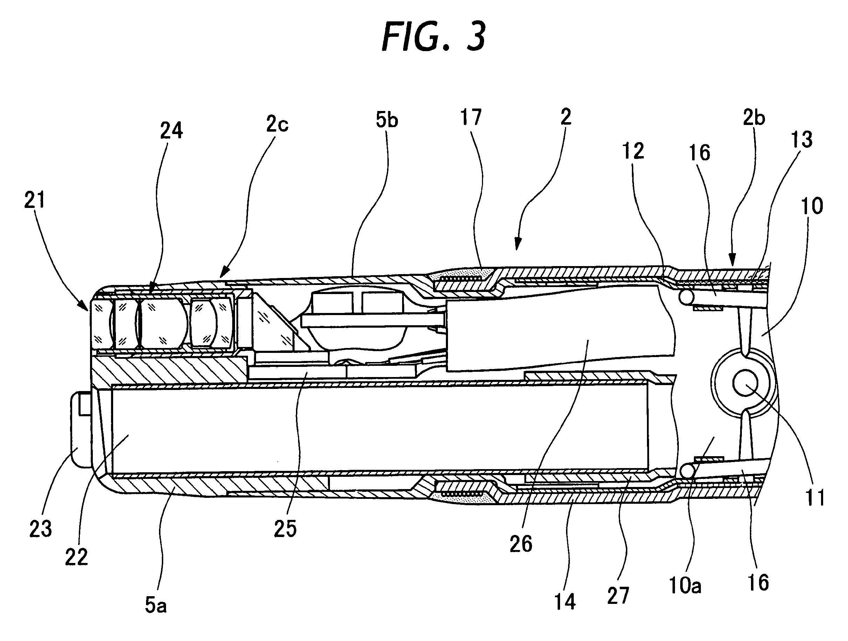

Fig. 2 shows in section a fore distal end portion of the endoscopic insertion tube, while Fig. 3 shows in section a joint portion between the articular section 2b and the flexible tube section 2a. As seen in these figures, the outside diameter of the endoscope is thickest in the articular section 2b and gradually reduced in the rigid tip end section 2c toward its fore distal end in a tapered fashion. On the other hand, in an intervening transitional section 2d between the articular section and the flexible tube section 2a, the outside diameter is gradually reduced in an inverse direction relative to the rigid tip end section 2c, namely, tapered off in a rearward direction. This transitional section 2d, with a rigid structure, is joined with the flexible tube section 2a at its proximal end which is tapered off to a maximum degree.

The articular section 2b is constituted by an articulated ring structure 12 consisting of a series of flexing rings 10 which are successively and pivotally connected by means of pivot pins 11. The articulated ring structure 12 is wrapped in a mesh tube 14 of knit wire fabric. Further, the mesh tube 14 is encased in an outer skin layer of elastic material like rubber. Thus, the articulated ring structure 12 is, so to say, a spinal structure of the articular section 2b, and a variety of component parts are threaded through the articulated ring structure. However, such threaded component parts as well as flexing wires are omitted in Fig. 2 for the convenience of illustration. In the particular embodiment shown, the articular section 2b can be flexed in either upward or downward direction, and for this purpose the respective flexing rings 10 are connected with an adjacent ring or rings by pivot pins 11 at articular joint portions at the opposite lateral sides of the respective rings.

For the purpose of threading flexing wires 16, each one of the flexing rings 10 is provided with drawn-out tubes 15 at top and bottom positions. The fore end of each flexing wire 16 is securely anchored on a foremost ring 10a (Fig. 3), after being threaded through the drawn-out tubes 15 in the arcitular section 2b. The proximal end of each flexing wire 16 is extended into the manipulating head grip 1 from the flexible tube section 2a, and wound on a pulley (not shown) which is mounted internally of a casing of the manipulating head grip 1 for rotation with a flexion control knob 4. In case the articular section 2b is to be flexed in four directions, i.e., in upward, downward, rightward and leftward directions, the flexing rings are pivotally joined with each other by pivot pins successively at top and bottom articular joints and at right and left articular joints as well. The four flexing wires are threaded through eyelets which are provided on the respective pivot pins.

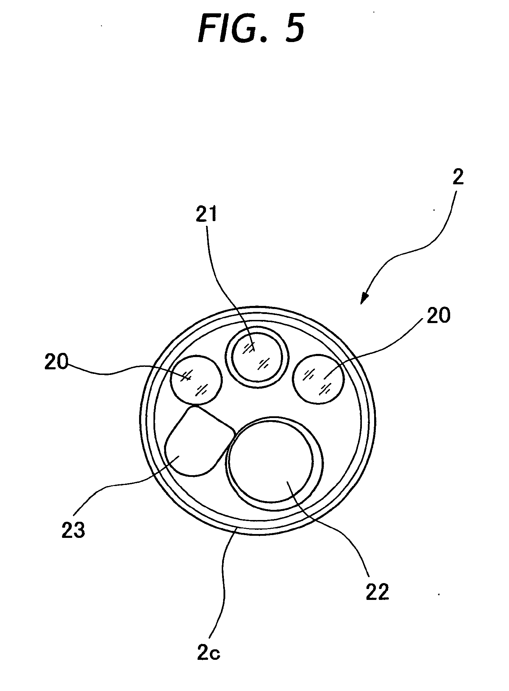

The rigid tip end section 2c which is connected to the fore end of the articular section 2b is largely composed of a casing 5a and a connector ring 5b, and, as shown in Fig. 5, a couple of illumination window 20 and an endoscopic observation window 21 are provide on this rigid tip end section 2c. The illumination windows 20 are located at opposite sides of the observation window 21. An outlet 22 of a tool deliver channel is opened at a position approximately beneath the observation window 21. On the other hand, a wash fluid jet nozzle 23 is located obliquely downward of the observation window 21, with a spout hole of the nozzle 23 pointed toward the observation window 21.

Thus, under the illuminant light from the illumination windows 20, an intracavitary site of interest is optically examined through the observation window 21. When a diseased portion is detected by the endoscopic observation through the observation window 21, a surgical instrument such as forceps or high frequency tool is led out from the outlet 22 of the tool delivery channel to give a suitable treatment to the diseased portion. For this purpose, it is necessary for an operator to get a clear view field constantly through the observation window 21, by washing the observation window 21 when contaminated with body fluids, with a wash liquid which is spurted out toward the observation window 21 from the jet nozzle 23, followed by blasting of compressed air to expel droplets and moisture.

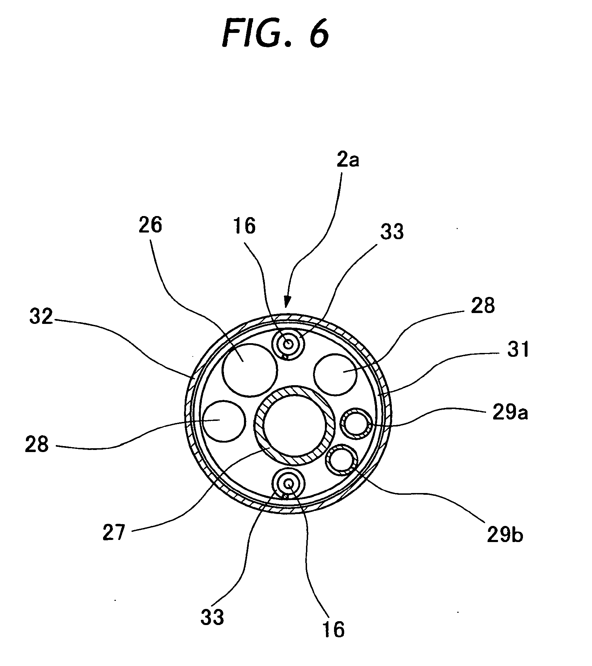

As seen in Fig. 3, a light guide is fitted in each one of the illumination windows 20 to project illuminant light toward an intracavitary site of interest, while an objective optical system is fitted in the observation window, including an image pick-up unit 25 having a solid state image sensor device located at the focus of the objective optical system. A cable, that is, a bundle of a predetermined number of signal lines, is led out form the image pick-up unit 25. Further, a tool delivery channel 27 from the manipulating head grip 1 is connected to the tool outlet 22, and a wash fluid conduit is connected to the injection nozzle 23. Although a light guide and a wash fluid conduit tube do not appear in a sectional view in the position of Fig. 3, a light guide is shown at 28 in Fig. 6, along with an air feed tube 29a and a water feed tube 29b which jointly constitute the above-mentioned wash fluid conduit. Thus, at least a light guide 28, a bundling cable, tool delivery channel 27 and air feed tube 29a and water feed tube 29b of the wash fluid conduit from the manipulating head grip 1 need to be internally threaded through the articular section 2b and the elongated flexible tube section 2a.

The rigid tip end section 2c which is composed of a rigid tubular casing 5a and a connector ring 5b which is joined with a foremost flexing ring 10a of the articular section 2b. The outer skin layer 14 is fitted around and fixed on a proximal end portion of the connector ring 5a by a fastener means 17 composed of line wrapping and a cementing adhesive agent. The diameter of the endoscopic insertion tube is increased to a maximum degree at this fastener means 17 and gradually reduced from that portion along the entire length of the rigid tip end section 2c as far as a fore distal end face of the latter. In this manner, the endoscopic insertion tube is arranged to have a minimum diameter at a fore end face of the rigid tip end section 2c, with the illumination windows 20, endoscopic observation window 21, tool outlet 22 and injection nozzle 23 located in a concentrated manner on a reduced surface area of the distal end face of the rigid tip section 2c.

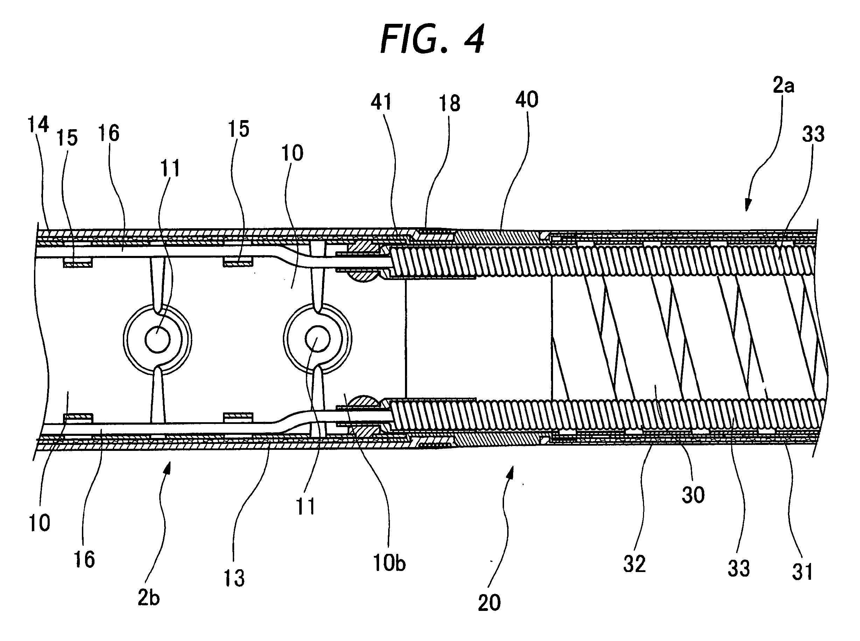

On the other hand, as shown in Fig. 4, the elongated flexible tube section 2a which is connected to the proximal end of the articular section 2b has a mesh tube 31 fitted on the double coil tube 30, a flexible structural member of the flexible tube section 2a. The mesh tube 31 is covered with an outer skin layer 32 which is laminated on the mesh tube 31, for example, by extrusion molding. Similarly to the articular section 2b, a number of component parts are threaded internally of the flexible tube section 2a of the insertion tube 2. The flexing wires 16 which are threaded through the drawn-out tubes 15 in the articular section 2b are passed through tightly wound sheathing coils in the flexible tube section 2a.

The articular section 2b can be flexed up to an angle of 180 degrees or more for the purpose of turning the endoscopic observation means on the rigid tip end section in a direction away from the advancing direction of the insertion tube 2. In contrast, the flexible tube section 2a cannot be bent through such a large angle. Therefore, the flexing rings 10 of the articulated ring structure 12, a flexible structural member of the articular section 2b, are formed in a greater thickness as compared with the double coil tube 40 of the flexible tube section 2a. Besides, since the articular section 2b is flexed through a large angle as mentioned above, the packing rate in this section needs to be suppressed to a minimum to secure an internal space which is broad enough for preventing jamming of threaded component parts which might take place on flexions through a large angle. In contrast, in the flexible tube section 2a which is not required to flex through a large angle, there is no necessity for suppressing the packing rate of the internally threaded component parts to such a degree as in the articular section 2b. That is to say, if necessary, the internally threaded component part may be packed, so to say, in a congested state in the flexible tube section 2a.

As discussed above, it is possible to step down the outside diameter of the flexible tube section 2a from the articular section 2b. That is to say, the flexible tube section 2a can be formed in a minimum necessary diameter irrespective of the outside diameter of the articular section. In that case, it becomes necessary to smoothen out the profile of the insertion tube, getting rid of adverse effects of a marked difference in outside diameter between the articular section 2b and the flexible tube section 2a as well as the existence of the fastener means 18 which is formed by applying line wrapping and an adhesive agent on an end portion of the outer skin layer 14 on the articular section 2b to add to the outside diameter portion of the insertion tube 2.

As shown in Fig. 4, a transitional section 2d in the form of a taper ring 40 is provided between the articular section 2b and the flexible tube section 2a. The taper ring 40 is provided with a short connecting sleeve 41 at its fore end for fitting engagement with a rearmost flexing ring of the articulated ring structure 12 of the articular section 2b. A proximal end portion of the outer skin layer 14 on the side of the articular section 2b is fitted on the connecting sleeve 41 of the taper ring 40, and securely fixed to the latter by line wrapping and an adhesive agent of a fastener means 18. Fore end of the double coil tube 30 is connected to a proximal end of the taper ring 40 of the transitional section 2d, serving as a foremost ring of the flexible tube section 2a. An outer skin layer 32 on the side of the flexible tube section 2a is extended forward as far as a halfway point on the taper ring 40.

In this instance, the outside diameter of the taper ring 40 is thickest at its fore end which is connected to the articular section 2b, and gradually tapered off toward its proximal end which is connected to the flexible tube section 2a. That is, the taper ring 40 is tapered in an inverse direction as compared with the rigid tip end section 2c. However, on the inside, the taper ring 40 has a uniform diameter along its entire length in the axial direction. Besides, the adhesive of the fastener means 18 is tapered off in a rearward direction in conformity with the taper angle of the taper ring 40 in such a way as to form a fore extension of the latter. On the other hand, a fastener means 18 at the proximal end of the taper ring 40, which is connected to the flexible tube section 2a, is in the shape of a moderate curve connecting the proximal end of the tapered transitional section 2d smoothly to the cylindrical flexible tube section 2a.

Since the taper ring 40 is a rigid member, a rigid part is formed between the rearmost flexing ring in the articulated ring structure of the articular section 2b and the proximal end of the taper ring 40 which is connected to the flexible tube section 2a. From the standpoint of maneuverability along an insertion passage, it is desirable that the rigid section be shortened in axial length. However, if shortened to a minimum axial length, the rigid transitional section 2d has to be tapered off at an acute angle. Therefore, the length and taper angle of the rigid transitional section 2d need to be set at suitable values in relation with internal conditions of insertion passages. For example, the transitional section 2d may be arranged to have substantially the same taper angle as the rigid tip end section 2c.

By adoption of the arrangements as described above, the endoscopic insertion tube 2 can be passed smoothly and easily even at a narrowly constricted part of a body cavity since the fore distal end of the rigid tip end section 2c acts to spread wider the constricted portion, lessening pains on the part of the examinee to a minimum. Besides, the maneuverability of the insertion tube 2 can be improved to a significant degree by the reduction in diameter of the rigid tip end section 2c, coupled with the reduction in length of the rigid section of the insertion tube 2. The smaller the diameter at the fore distal end of the rigid tip end section 2c, the higher becomes the performance of the rigid tip end section 2c in getting into and spreading wider a constricted part in an insertion passage. For this reason, the illumination windows 20, observation window 21 and tool outlet hole 23 are located in a concentrated manner in a reduced surface area on the distal end face of the rigid tip end section 2c.

In the case of a per-nasal endoscope, after introduction into the nasal cavity from the anterior naris, it has to be passed through a constriction in a middle passage which is surrounded by the nasal septum, middle turbinate and inferior turbinate. Because of differences between individual examinees, an endoscopic insertion tube of a certain outside diameter which is passable through such a passage in the nasal cavity of one person without meeting a large resistance may become impassable through a similar passage in the nasal cavity of other person due to a stubborn resistance which makes the further passage of the insertion tube impossible or very difficult if not impossible. However, since the fore end portion of the insertion tube 2 is tapered off in the forward direction, it can be advanced into a constricted part in an insertion passage smoothly in a secure manner. Namely, depending upon the degree of constriction of an insertion passage, a constricted part can be spread wider by the forwardly tapered rigid tip end section 2c to let the following parts of the insertion tube 2 pass smoothly through the constricted part of the insertion passage.

In this connection, a constricted part in an insertion passage is spread to a maximum degree upon passage of the articular section 2b. After passage of the articular section 2b, the flexible tube section 2a comes to the constricted part. Upon passage of the flexible tube section 2a which is appreciably thinner than the articular section 2b, the spreading force which has been acting on the constricted part is taken off or lessened to a certain degree, permitting the constricted part to return almost to a natural state almost free of a spreading force. Accordingly, oppressive sensations are lessened to a significant degree to relieve an examinee from pains. The insertion tube 2 is then advanced smoothly up to an entrance to the esophagus or into the esophagus via the coana. Now, it is only the narrower flexible tube section 2a which remains in the constricted part, so that the insertion passage is almost freed from oppressions by the spreading force.

Upon completion of an endoscopic examination, the insertion tube 2 is pulled out of the insertion passage in the nasal cavity. At this time, the transitional section 2d between the flexible tube section 2a and the articular section 2c is shifted back to the position of the constricted part. Since the transitional section 2d is tapered off in the inverse direction, the constricted part is gradually spread wider by the transitional section 2d again to such an extent as to permit passage of the articular section 2b. Thus, the endoscopic insertion tube 2 can be inserted into and out of a passage in the nasal cavity very smoothly.

Due to a necessity arising from a peculiar internal construction and for protection of internally threaded parts including flexing movements of flexing parts, the articular section 2b is necessarily required to have a sufficient thickness in outside diameter unlike other sections of the insertion tube. However, the flexible tube section 2a on the proximal side of the articular section 2b can be uniquely downsized in outside diameter irrespective of the outside diameter of the articular section 2b. In that case, even though the flexibe tube section 2a is appreciably reduced in diameter as compared with the articular section 2b, it has no possibility of causing damages to internally threaded component parts at all. Thus, the maneuverability of the endoscopic insertion tube 2 at a constricted part of an insertion passage can be improved significantly, while lessening burdens and pains on the part of an examinee to a considerable degree.

## Claims

1. An endoscope having an insertion tube (2) extended out of a manipulating head grip (1), said insertion tube (2) having in series an elongated flexible tube section (2a), an articular section (2b) and a rigid tip end section (2c) from a proximal end connected to said manipulating head grip (1), wherein
said rigid tip end section (2c) is tapered off in a forward direction gradually toward a fore distal end from a proximal end connected with said articular section (2b) and provided with at least an illumination window (20) and an observation window (21) on a fore distal end face; and
an articular section (2b) having a larger outside diameter than any other section of said insertion tube (2) ; **characterised by**
a transitional section (2d) tapered off in an inverse direction as compared with said rigid tip end section (2c), gradually from a fore end on the side of said articular section (2b) toward a proximal end on the side of said flexible tube section (2a).

2. An endoscope as set forth in claim 1, wherein said articular section (2b) and said flexible tube section (2a) of said insertion tube (2) are formed in predetermined outside diameters, respectively, and are intervened by said transitional section (2d) in the form of a rigid taper ring (40).

3. An endoscope as set forth in claim 2, wherein said taper ring (40) is gradually tapered off toward a proximal end thereof on the outer side, and formed with a through axial hole of a uniform diameter on the inner side.

4. An endoscope as set forth in claim 1, wherein said rigid tip end section (2c) and said transitional section (2d) are tapered off with substantially the same taper angle but in inverse directions relative to each other.

5. An endoscope as set forth in claim 1, further comprising an outer skin layer (14) fitted on said articular section and fixedly anchored at opposite ends on said rigid tip end section (2c) and said transitional section, respectively, by line wrapping and an adhesive agent of a fastener means in conformity with tapered shapes of said rigid tip end section (2c) and said transitional section 2d.

## Patentansprüche

1. Endoskop mit einem sich aus einem Manipulierhandgriff (1) heraus erstreckenden Einführschlauch (2), der einen länglichen flexiblen Schlauchabschnitt (2a); einen Gelenkabschnitt (2b) und einen starren Endabschnitt (2c) in der Reihenfolge von einem an dem Manipulierhandgriff (1) angeschlossenen proximalen Ende aufweist, wobei
der starre Endabschnitt (2c) in einer Vorwärtsrichtung von einem mit dem Gelenkabschnitt (2b) verbundenen proximalen Ende zu einem distalen Ende allmählich verjüngt ist und mit mindestens einem Beleuchtungsfenster (20) und einem Betrachtungsfenster (21) an einer vorderen distalen Stirnfläche ausgestattet ist; und ein Gelenkabschnitt (2b) mit einem größeren Außendurchmesser als jeder andere Abschnitt des Einführschlauchs (2) ausgestattet ist, **gekennzeichnet durch** einen Übergangsabschnitt (2d), der im Vergleich zu dem starren Endabschnitt (2c) in entgegengesetzte Richtung von einem Vorderende auf der Seite des Gelenkabschnitts (2b) in Richtung eines proximalen Endes auf der Seite des flexiblen Schlauchabschnitts (2a) allmählich verjüngt ist.

2. Endoskop nach Anspruch 1, bei dem der Gelenkabschnitt (2b) und der flexible Schlauchabschnitt (2a) des Einführschlauchs (2) mit vorbestimmten Außendurchmessern ausgebildet sind, wobei der Übergangsabschnitt (2d) in Form eines starren konischen Rings (40) dazwischen liegt.

3. Endoskop nach Anspruch 2, bei dem der konische Ring (40) auf der Außenseite allmählich zu einem proximalen Ende hin verjüngt ist und mit einem axialen Durchgangsloch gleichförmigen Durchmessers auf der Innenseite ausgebildet ist.

4. Endoskop nach Anspruch 1, bei dem der starre Endabschnitt (2c) und der Übergangsabschnitt (2d) mit im wesentlichen demselben Verjüngungswinkel, jedoch in einander entgegengesetzte Richtungen verjüngt sind.

5. Endoskop nach Anspruch 1, weiterhin umfassend eine Außenhautschicht (14), die auf den Gelenkabschnitt aufgepasst und an einander entgegengesetzten Enden an dem starren Endabschnitt (2c) und dem Übergangsabschnitt durch Lagenwicklung und an Klebstoff einer Befestigungseinrichtung konform zu den Verjüngungen des starren Endabschnitts (2c) und des Übergangsabschnitts (2d) verankert ist.

## Revendications

1. Endoscope ayant un tube d'insertion (2) qui s'étend depuis un manche de tête de manipulation (1), ledit tube d'insertion (2) ayant en série une section de tube flexible allongé (2a), une section articulaire (2b) et une section d'extrémité de bout rigide (2c) depuis une extrémité proximale reliée audit manche de tête de manipulation (1), où ladite section d'extrémité de bout rigide (2c) est rétrécie dans une direction avant progressivement en direction d'une extrémité distale antérieure depuis une extrémité proximale reliée à ladite section articulaire (2b) et munie d'au moins une fenêtre d'illumination (20) et une fenêtre d'observation (21) sur une face terminale distale antérieure ; et
une section articulaire (2b) ayant un plus grand diamètre externe que toute autre section dudit tube d'insertion (2) ; **caractérisé par**
une section de transition (2d) rétrécie dans une direction inverse par rapport à ladite section d'extrémité de bout rigide (2c), progressivement depuis une extrémité antérieure sur le côté de ladite section articulaire (2b) en direction d'une extrémité proximale sur le côté de ladite section de tube flexible (2a).

2. Endoscope selon la revendication 1, où ladite section articulaire (2b) et ladite section de tube flexible (2a) dudit tube d'insertion (2) sont formées en des diamètres externes prédéterminés, respectivement, et sont séparées par ladite section de transition (2d) sous forme d'un anneau rétréci rigide (40).

3. Endoscope selon la revendication 2, où ledit anneau rétréci (40) est rétréci progressivement en direction d'une extrémité proximale de celui-ci sur le côté externe, et formé avec un trou axial traversant d'un diamètre uniforme sur le côté interne.

4. Endoscope selon la revendication 1, où ladite section d'extrémité de bout rigide (2c) et ladite section de transition (2d) sont rétrécies avec sensiblement le même angle de rétrécissement mais dans des directions inverses l'une par rapport à l'autre.

5. Endoscope selon la revendication 1, comprenant en outre une couche de peau externe (14) disposée sur ladite section articulaire et ancrée de manière fixe à des extrémités opposées sur ladite section d'extrémité de bout rigide (2c) et ladite section de transition, respectivement, par enveloppement linéaire et un agent adhésif d'un moyen de fixation en conformité avec les formes rétrécies de ladite section d'extrémité de bout rigide (2c) et de ladite section de transition (2d).
